(19) Europäisches Patentamt

European Patent Office

Office européen des brevets

(11) **EP 1 466 553 B1**

(12) **EUROPEAN PATENT SPECIFICATION**

(45) Date of publication and mention
of the grant of the patent:
**02.07.2008 Bulletin 2008/27**

(51) Int Cl.:
*A61B 3/11* *(2006.01)*

(21) Application number: **04101480.4**

(22) Date of filing: **09.04.2004**

(54) **Automatic pupillometer**

Automatisches Pupillometer

Pupillomètre automatique

(84) Designated Contracting States:
**DE ES FR GB IT**

(30) Priority: **10.04.2003 IT MI20030740**

(43) Date of publication of application:
**13.10.2004 Bulletin 2004/42**

(73) Proprietor: **Ligi Tecnologie Medicali S.p.A.**
**74100 Taranto (IT)**

(72) Inventors:
• **D'Ippolito, Giuseppe**
**74020 S.Vito (IT)**

• **Lipari, Eugenio**
**98021 Ali Terme (IT)**
• **Rubino, Anna Rosa**
**72022 Latiano (IT)**
• **Basetti, Giovanni**
**74100 Taranto (IT)**

(74) Representative: **Jorio, Paolo et al**
**Studio Torta S.r.l.**
**Via Viotti, 9**
**10121 Torino (IT)**

(56) References cited:
**EP-A- 1 127 534** **US-B1- 6 199 985**

**Description**

The present invention refers to an automatic pupillometer.

As is known, pupillometers are commonly used in ophthalmology to measure the pupillary diameter of a patient. Currently available pupillometers, such as described in EP 1 127 534 A, are equipped with a chamber, which is suitable for coupling to one or both eyes of a patient, with a light source arranged inside the chamber and supplying infrared and visible radiation of predetermined intensities, and with an apparatus for the acquisition of images, sensitive to both visible and infrared radiation. The portion of the chamber intended for connection to the eye of the patient is essentially lightproof, i.e. is shaped in such a way as to prevent light entering the chamber, from the external environment, while measurements are being taken. Such measurements, in fact, must be performed in predetermined lighting conditions which are as reproducible as possible, since, as is known, the diameter of the pupil varies according to the intensity of incident light radiation. It is clear that a contribution of light coming from the surrounding environment would be added to the light present in the chamber, thus unpredictably altering the conditions to which the eye is subjected and making the measurement less reliable. Normally, the pupillary diameter is measured in two conditions. In a first case (the so-called photopic condition), the light source emits a light beam of predetermined and rather high intensity towards the eye of the patient: the pupil therefore contracts to its minimum aperture. In a second case (scotopic conditions), the chamber is basically deprived of visible lighting and therefore the pupil dilates to its maximum aperture.

The known pupillometers suffer from certain drawbacks. Firstly, the measurable information concerning the pupillary dynamics is rather limited. In fact, as mentioned above, conventional pupillometers measure the pupillary diameter only in two borderline cases, which the patient only rarely experiences during usual daily activities. Instead, it is not possible to know the pupillary diameter in the most frequent lighting conditions for the patient (i.e., in the lighting conditions to which the patient is most often subjected during daily activities). More detailed information on the pupillary dynamics would instead be extremely important, for example, to perform photoablative corneal surgery. In fact, one of the problems connected with this type of intervention concerns the balancing of two conflicting requirements, connected with the amplitude of the field of vision and therefore of the pupillary diameter in different circumstances. On the one hand, the corneal area to be treated should be the widest possible, so as to avoid irregularities within the field of vision in any lighting conditions. On the other hand, the ablation of an excessive quantity of corneal tissue may involve some serious risks; it may be expedient to limit intervention to an area mainly concerning vision, and allowing irregularity of the cornea in a marginal region of the field of vision which is rarely used by the patient.

Another disadvantage of known pupillometers is that the lighting conditions can change if connection to the eyes of the patient is not optimum and therefore some external light can filter into the chamber; in some cases, even, it is necessary to reduce or virtually eliminate the environmental light. Similarly, the lighting conditions are altered if the light supplied from the light source differs in intensity to that of the envisaged nominal intensity.

The purpose of the present invention is to realize an automatic pupillometer, which does not have the described drawbacks.

An automatic pupillometer is realized according to the present invention as claimed in claim 1.

In this way, it is possible to measure the pupillary diameter in different lighting conditions, which correspond to as many situations which patients experience during their usual activities. The coefficients associated with each selected intensity value can be used to "weight" the different lighting conditions according to the activity of the subject: for instance, a high coefficient is associated with an intensity value corresponding to a condition frequently experienced by the patient. Instead, if an intensity value corresponds to a condition which the patient experiences for only a short time, then a smaller coefficient is associated with this intensity value. The pupillometer according to the invention therefore allows information to be extracted concerning the amplitude of the field of vision predominantly used by the subject. In addition, the measurement is objective and is carried out quickly, thus avoiding alterations caused by fatigue in the subject.

For a better understanding of the invention, an embodiment of it is now described as follows, being a purely illustrative, non-limiting description which refers to the enclosed figures, in which:

- figure 1 is a block diagram of an automatic pupillometer realized according to the present invention;
- figure 2 schematically illustrates an eye of a subject;
- figure 3 is a graph that illustrates magnitudes relating to the operation of the pupillometer of figure 1;
- figures 4 and 5 are more detailed block diagrams of parts of the pupillometer of figure 1; and
- figure 6 is a graph that illustrates magnitudes relating to the operation of the pupillometer of figure 1.

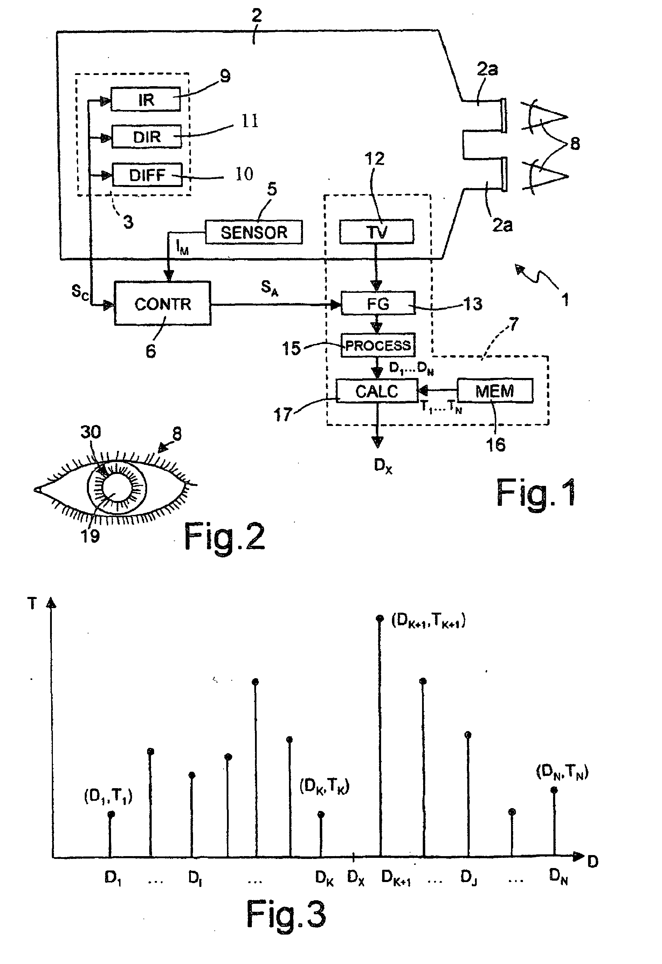

With reference to figure 1, an automatic pupillometer is denoted by number 1, which includes a chamber 2, a lighting device 3, a sensor 5 of light intensity, a control device 6, cooperating with the sensor 5 to control the lighting device 3, and a measurement apparatus 7 of the pupillary response. The chamber 2 is connectable, by means of eyepieces 2a, to at least one and preferably both eyes 8 of a subject undergoing an examination. Furthermore, the connection is lightproof, i.e. it does not allow light from the surrounding environment to penetrate inside the chamber 2.

The lighting device 3 includes at least one infrared light source 9 and a visible light source 10, supplying diffused light stimuli having selectable intensities $I_1, I_2, ..., I_N$ (N is the number of light stimuli sent during the examination of a subject). In the non-limiting embodiment described, the lighting device 3 also has a visible light source 11 supplying direct light stimuli (i.e. is directly focussed towards the eyes 8 of the subject), this also having selectable intensities $I_1, I_2, ..., I_N$. Preferably, also, the sources 10 and 11 include a plurality of LEDs having different emission frequencies and which are separately controllable.

The control device 6 has an input connected to the sensor 5, to receive a measurement signal $I_M$, correlated with an actual value of light intensity incident on the sensor 5, and an output, connected to the lighting device 3 and supplying a control signal $S_c$, correlated with a selected value of intensity $I_1, I_2, ..., I_N$ and with the measurement signal $I_M$. In practice, the control device 6, by the control signal $S_c$, controls the lighting apparatus 3 so as to maintain conditions of nominal lighting for each of the selectable values of intensity $I_1, I_2, ..., I_N$ of the light stimuli.

The measurement apparatus 7 of the pupillary response includes a camera 12, a converter 13 ("frame grabber"), an image processing unit 15, a memory 16 and a calculation unit 17. The camera 12, of analogue type here, is sensitive to both visible radiation and infrared radiation and is arranged so as to take images of the eyes 8 of the subject, particularly the pupils 19 (figure 2). The camera 12 provides the converter 13 with a video signal $S_V$, representative of the images taken. Furthermore, in a preferred embodiment of the invention, the sensor 5 is integrated into the chamber 2.

The converter 13 samples the video signal $S_V$ and is controlled by an activation signal $S_A$ supplied by the control unit 6 for storing and supplying sequences of samples of the video signal $S_V$, corresponding to photograms, to the image processing unit 15 (digitized images).

The image processing unit 15 processes the digitized images to determine the values of a plurality of characteristic parameters of the pupils 19 of the subject in the actual lighting conditions. More precisely, starting with each digitized image, the image processing unit 15 determines values of an average pupillary diameter $D_1, D_2, ..., D_N$, (the pupils 19 are not perfectly circular) and, preferably, of other parameters such as maximum and minimum pupillary diameters, an angle between the directions of the maximum and minimum pupillary diameters and the position of the pupillary centroid; obviously, the values of the parameters and, in particular, the values of the average pupillary diameter $D_1, D_2, ..., D_N$, correspond to respective intensity values $I_1, I_2, ..., I_N$ of the light stimuli, i.e. to respective lighting conditions; for instance, if the intensity values $I_1, I_2, ..., I_N$ are decreasing, the corresponding average pupillary diameters $D_1, D_2, ..., D_N$, are increasing. The calculation unit 17 is provided with the values of the parameters thus determined.

The memory 16 stores weight coefficients $T_1, T_2, ..., T_N$, each associated with a respective of the values of intensity $I_1, I_2, ..., I_N$ of the light stimuli. In particular, each of the coefficients $T_1, T_2, ..., T_N$ is representative of the average time spent by the subject in carrying out his/her usual activities in a lighting condition corresponding to an intensity value $I_1, I_2, ..., I_N$ of the light stimuli.

The coefficients $T_1, T_2, ..., T_N$ and the values of the average pupillary diameter $D_1, D_2, ..., D_N$, are supplied to the calculation unit 17, which determines an optimum pupillary diameter $D_X$. In practice, the optimum pupillary diameter $D_X$ is calculated by considering the lighting conditions that occur most frequently for the subject. The weight attributed to each light condition is represented by the relative coefficient $T_1, T_2, ..., T_N$. In the non-limiting embodiment described, the optimum pupillary diameter $D_X$ is calculated so that the following conditions are satisfied simultaneously (see also figure 3):

$$(100 - P)\sum_{I=1}^{K} T_I(D_X - D_I) = P \sum_{J=K+1}^{N} T_J(D_J - D_X) \qquad (1)$$

$$D_X \in [D_K, D_{K+1}] \qquad (2)$$

where N is the number of light stimuli generated by the lighting device 3, K is an integer number between 2 and N-1 and P is a percentage coefficient between 0 and 100. In practice, the percentage coefficient P indicates the percentage of the events relating to the daily activities of the subject in which the average pupillary diameter $D_1, D_2, ..., D_N$, is less than the optimum pupillary diameter $D_X$; in other words, in the daily activities of the subject, events in which the average pupillary diameter $D_1, D_2, ..., D_N$ is greater than the optimum pupillary diameter $D_X$ have an occurrence probability of 100-P. In practice, the average pupillary diameter is less than the optimum pupillary diameter $D_X$ basically in all the statistically significant conditions in the daily activities of the subject and therefore the ideal pupillary diameter $D_X$ is indicative of the portion of corneal area predominantly used by the subject.

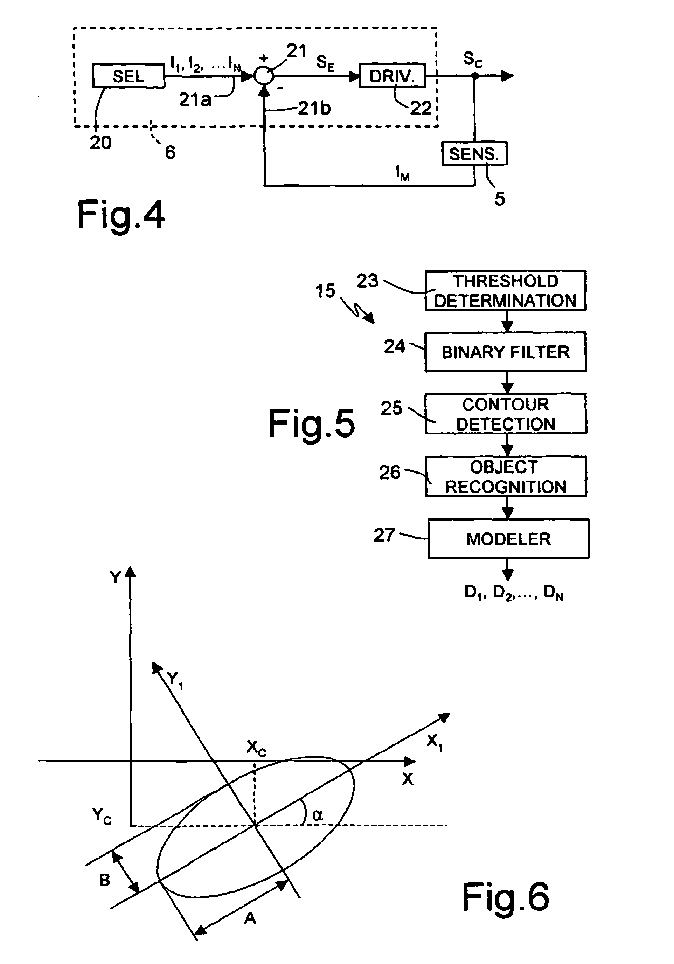

With reference to figures 1 and 4, the control device 6 includes a selector 20 of intensity, a subtracting node 21 and a driving unit 22. In greater detail, the selector 20 sequentially selects one of the values of intensity $I_1, I_2, ..., I_N$

of the light stimuli and supplies a non-inverting input 21a of the subtracting node 21 with it. The subtracting node 21 besides has an inverting input 21b connected to the sensor 7, to receive the measurement signal $I_M$, and an output connected to the driving unit 22 and supplying an error signal $S_E$, which is correlated with the difference between the selected value of intensity $I_1$, $I_2$, ..., $I_N$ and the measurement signal $I_M$ (the subtracting node acts, in practice, as a comparator stage). In turn, the driving unit 22 determines and supplies the control signal $S_C$ to the lighting device 3, for modifying the intensity of the light radiation emitted so as to cancel the error signal $S_E$. Owing to the control device 6, the level of light inside the chamber 2 can be kept constant and basically the same as the nominal conditions envisaged for each selectable intensity value $I_1$, $I_2$, ..., $I_N$. In particular, measurement errors, due to light coming from the external surroundings because of a defective connection to the eyes 8 of the subject, may be prevented; in this case, the control device can reduce the intensity of the radiation emitted by the lighting device to compensate for the contribution due to environmental light. Preferably, the control device 6 is configured for generating an enabling signal, when the lighting conditions inside the chamber 2 correspond to the nominal conditions for the selected intensity value $I_1$, $I_2$, ..., $I_N$, and an inhibiting signal, which prevents the measurement being taken if the nominal conditions are not reached.

As shown in figure 5, the image processing unit 15 includes a threshold determination block 23, a binary filter 24, a contour detection block 25, an object recognition block 26 and a modeller 27, which are cascade-connected.

In detail, the threshold determination block 23, from the digitized image received in input, establishes a first sample region, inside one of the pupils 19, and a second sample region, inside the iris of the corresponding eye 8, and calculates an average brightness value $L_P$ of the first sample region and an average brightness value $L_I$ of the second sample region. Then, a brightness threshold $L_S$ is determined based on the relation:

$$L_S = L_P + Q(L_I - L_P) \tag{3}$$

where Q is a percentage factor.

The binary filter 24 assigns a first or a second binary value to each point of the image alternatively, depending on whether the brightness value associated with the point is respectively lower or greater than the brightness threshold $L_S$. In practice, the first binary value is assigned to all points of the digitized image inside the pupils 19.

Then, the contour detection block 25 extracts the outlines of the portions of the digitized image which are identified by the first binary value, and the object recognition block 26 selects the points belonging to the pupillary contours 30 of the pupils 19 (figure 2).

The modeller 27 utilizes a second descriptive function order, expressed in parametric form, to describe the pupillary contours 30. More precisely, the modeller 27 is based on a descriptive function of type (ellipse, see figure 6):

$$A_{11}Y^2 + A_{22}X^2 + 2A_{12}XY + 2A_{13}X + 2A_{23}Y + A_{33} = 0 \tag{4}$$

where
$A_{11} = A^2 \sin \alpha^2 + B^2 \cos \alpha^2$;
$A_{22} = A^2 \cos \alpha^2 + B^2 \sin \alpha^2$;
$2A_{12} = (B^2 - A^2) \sin 2\alpha$;
$2A_{13} = - (2A_{11}X_C + 2A_{12} Y_C)$;
$2A_{23} = - (2A_{22} Y_C + 2A_{12} X_C)$;
$A_{33} = X_C{}^2 A_{11} + Y_C{}^2 A_{22} + X_C Y_C 2A_{12} - A^2B^2$
and $X_C$, $Y_C$ are the coordinates of the centre of the ellipse, A and B are the greater semiaxes and, respectively, smaller and $\alpha$ is the angle of rotation of the greater semiaxis A as regards the X axis. The modeller 27, by selecting values, in a way in itself known, of the descriptive parameters (for instance using the Levemberg-Marquardt method), determines the descriptive function which best approximates the pupillary contours 30 supplied by the blocks 25 and 26. Based on the descriptive function identified, lastly, the processing unit 15 extracts the values of the average diameter $D_1$, $D_2$, ..., $D_N$, of the maximum and minimum diameters and of the pupillary centroid.

Finally, it is clear that modifications and variations can be made to the pupillometer described, without leading away from the scope of the present invention. For example, the optimum pupillary diameter could be calculated starting with the average diameters $D_1$, $D_2$, ..., $D_N$, and with the coefficients $T_1$, $T_2$, ..., $T_N$ based on conditions which differ from the conditions (1) and (2) described; in particular, the optimum pupillary diameter could be calculated as a fraction of a weighted average of the average diameters $D_1$, $D_2$, ..., $D_N$. Also the function used to model the pupillary contour could be different from that which is described.

**Claims**

1. Automatic pupillometer, including:

a lightproof chamber (2), connectable to at least one eye (8) of a subject;
a lighting device (3), for supplying light stimuli having respective selectable intensity values ($I_1$, $I_2$, ..., $I_N$) inside the chamber (2);
pupillary response measurement means (7), to determine values of at least one pupillary diameter ($D_1$, $D_2$, ..., $D_N$,), said values corresponding to respective selected intensity values ($I_1$, $I_2$, ..., $I_N$);
**characterized in that** the pupillary response measurement means (7) include storage means (16), to store a group of coefficients ($T_1$, $T_2$, ..., $T_N$) associated with respective intensity values ($I_1$, $I_2$, ..., $I_N$), and calculation means (17), to calculate an optimum pupillary diameter ($D_X$) based on the values of pupillary diameter ($D_1$, $D_2$, ..., $D_N$,) and the stored coefficients ($T_1$, $T_2$, ..., $T_N$).

2. Pupillometer according to claim 1, **characterized in that** said optimum value ($D_X$) satisfies the conditions:

$$(100 - P)\sum_{I=1}^{K} T_I(D_X - D_I) = P \sum_{J=K+1}^{N} T_J(D_J - D_X)$$

$$D_X \in \left[ D_K, D_{K+1} \right]$$

where N is the number of light stimuli generated by the lighting device (3), K is a whole number between 2 and N-1 and P is a percentage coefficient between 0 and 100.

3. Pupillometer according to in claim 1, **characterized in that** said optimum pupillary diameter ($D_X$) is correlated with a weighted average of said values of pupillary diameter ($D_1$, $D_2$, ..., $D_N$, ) based on the stored coefficients ($T_1$, $T_2$, ..., $T_N$).

4. Pupillometer according to any one of the foregoing claims, **characterized in that** said coefficients ($T_1$, $T_2$, ..., $T_N$) are correlated with average stay times of the subject in lighting conditions corresponding to respective intensity values ($I_1$, $I_2$, ..., $I_N$) of the light stimuli .

5. Pupillometer according to one of the foregoing claims, **characterized in that** said pupillary response measurement means (7) include image acquisition means (12, 13), for acquiring a respective image of the eye (8) for each selected intensity value ($I_1$, $I_2$, ..., $I_N$), and image processing means (15), associated with the image acquisition means (12, 13) for determining values of said diameter ($D_1$, $D_2$, ..., $D_N$, ) of the pupil (19) .

6. Pupillometer according to any one of the foregoing claims, **characterized in that** it comprises control means (6) of the lighting device, for controlling the intensity values ($I_1$, $I_2$, ..., $I_N$) of the light stimuli.

7. Pupillometer according to in claim 6, **characterized in that** the control means (6) include a selection unit (20) to select said intensity values ($I_1$, $I_2$, ..., $I_N$) of the light stimuli .

8. Pupillometer according to in claim 7, **characterized in that** it comprises a sensor (5) of light intensity, supplying a measurement signal ($I_M$) correlated with an existing intensity value of light radiation incident on the sensor (5), and **in that** the control means (6) comprise a comparison stage (21), supplying an error signal ($S_E$) correlated with a difference between the measurement signal ($I_M$) and the selected intensity value ($I_1$, $I_2$, ..., $I_N$), and a driving unit (22), associated with said lighting device (3) to modify the intensity value ($I_1$, $I_2$, ..., $I_N$) of the light stimuli based on the error signal ($S_E$), so as to cancel the error signal ($S_E$).

9. Pupillometer according to claim 8 dependent on claim 5, **characterized in that** the sensor (5) of light intensity is integrated into said chamber (2).

10. Pupillometer according to any one of the foregoing claims, **characterized in that** said lighting device (3) includes a diffused light source (10) and a direct light source (11).

**11.** Pupillometer according to any one of the claims 5 to 10, **characterized in that** said image processing means (15) include at least one binary filter (24), a contour detection unit (25), for identifying a pupillary contour, and a modeller (27), to determine a function approximating the pupillary contour.

**12.** Pupillometer according to claim 11, **characterized in that** the approximating function is a second order function.

**Patentansprüche**

**1.** Automatisches Pupillometer, umfassend:

eine lichtdichte Kammer (2), die mit mindestens einem Auge (8) eines zu untersuchenden Subjekts verbindbar ist; eine Beleuchtungseinrichtung (3), um in der Kammer (2) Lichtstimuli mit den entsprechenden auswählbaren Intensitätswerten ($I_1$, $I_2$, ..., $I_N$) zu liefern; Mittel (7) zum Messen der Pupillenreaktion, um die Werte von zumindest einem Pupillendurchmesser ($D_1$, $D_2$, ..., $D_N$,) zu bestimmen, wobei die Werte den zugehörigen ausgewählten Intensitätswerten ($I_1$, $I_2$, ..., $I_N$) entsprechen; **dadurch gekennzeichnet, dass** die Mittel (7) zum Messen der Pupillenreaktion Speichermittel (16) enthalten, um eine Gruppe von mit den Intensitätswerten ($I_1$, $I_2$, ..., $I_N$) verbundenen Koeffizienten ($T_1$, $T_2$, ..., $T_N$) zu speichern, und Berechnungsmittel (17), um den optimalen Pupillendurchmesser ($D_x$) auf der Basis des Pupillendurchmessers ($D_1$, $D_2$, ..., $D_N$,) und des gespeicherten Koeffizienten ($T_1$, $T_2$, ..., $T_N$) zu berechnen.

**2.** Pupillometer gemäß Anspruch 1, **dadurch gekennzeichnet, dass** der optimale Wert ($D_x$) die Bedingungen erfüllt:

$$(100 - P)\sum_{i=1}^{K} T_i (D_x - D_i) = P \sum_{j=K+1}^{N} T_j (D_j - D_x)$$

$$D_x \in \left[ D_K, D_{K+1} \right]$$

wobei N die Anzahl der Lichtstimuli ist, die durch die Beleuchtungseinrichtung (3) erzeugt werden, K eine Ganzzahl zwischen 2 und N-1 und P ein prozentualer Koeffizient zwischen 0 und 100.

**3.** Pupillometer gemäß Anspruch 1, **dadurch gekennzeichnet, dass** der optimale Pupillendurchmesser ($D_x$) mit einem gewichteten Durchschnitt der Pupillendurchmesserwerte ($D_1$, $D_2$, ..., $D_N$,) basierend auf den gespeicherten Koeffizienten ($T_1$, $T_2$ ..., $T_N$) korreliert wird.

**4.** Pupillometer gemäß einem der vorhergehenden Ansprüche, **dadurch gekennzeichnet, dass** die Koeffizienten ($T_1$, $T_2$, ..., $T_N$) mit den durchschnittlichen Verweilzeiten der zu untersuchenden Person unter den Beleuchtungsbedingungen entsprechend den jeweiligen Intensitätswerten ($I_1$, $I_2$, ..., $I_N$) der Lichtstimuli korreliert werden.

**5.** Pupillometer gemäß einem der vorhergehenden Ansprüche, **dadurch gekennzeichnet, dass** die Mittel (7) zum Messen der Pupillenreaktion ein Bilderfassungsmittel (12,13) enthalten, um ein entsprechendes Abbild des Auges (8) für jeden ausgewählten Intensitätswert ($I_1$, $I_2$, ..., $I_N$) zu erfassen, und Bildverarbeitungsmittel (15), die mit den Bilderfassungsmitteln (12, 13) verknüpft sind, um die Durchmesserwerte ($D_1$, $D_2$, ..., $D_N$,) der Pupille (19) zu bestimmen.

**6.** Pupillometer gemäß einem der obigen Ansprüche, **dadurch gekennzeichnet, dass** es Steuermittel (6) für die Beleuchtungseinrichtung enthält, um die Intensitätswerte ($I_1$, $I_2$, ..., $I_N$) der Lichtstimuli zu steuern.

**7.** Pupillometer gemäß Anspruch 6, **dadurch gekennzeichnet, dass** die Steuermittel (6) eine Auswahleinheit (20) umfassen, um die Intensitätswerte ($I_1$, $I_2$, ..., $I_N$) der Lichtstimuli auszuwählen.

**8.** Pupillometer gemäß Anspruch 7, **dadurch gekennzeichnet, dass** es einen Sensor (5) für die Lichtintensität umfasst, der ein Messsignal ($I_M$) liefert, das mit einem bestehenden Intensitätswert der auf den Sensor (5) einfallenden Lichtstrahlung korreliert wird, und darin, dass die Steuermittel (6) eine Vergleichsstufe umfassen (21), wobei ein Fehlersignal ($S_E$) geliefert wird, das mit einer Differenz zwischen dem Messsignal ($I_M$) und dem ausgewählten Intensitätswert ($I_1$, $I_2$, ..., $I_N$) korreliert wird, und eine Antriebseinheit (22), die mit der Beleuchtungseinrichtung (3) verknüpft ist, um den Intensitätswert ($I_1$, $I_2$, ..., $I_N$) der Lichtstimuli basierend auf dem Fehlersignal ($S_E$) zu ändern, um das Fehlersignal ($S_E$) zu stornieren.

**9.** Pupillometer gemäß Anspruch 8, abhängig von Anspruch 5, **dadurch gekennzeichnet, dass** der Sensor (5) für die Lichtintensität in die Kammer (2) integriert ist.

**10.** Pupillometer gemäß einem der vorhergehenden Ansprüche, **dadurch gekennzeichnet, dass** die Beleuchtungseinrichtung (3) eine indirekte Lichtquelle (10) und eine direkte Lichtquelle (11) umfasst.

**11.** Pupillometer gemäß einem der Ansprüche 5 bis 10, **dadurch gekennzeichnet, dass** die Bildverarbeitungsmittel (15) zumindest einen Binärfilter (24), eine Konturerfassungseinheit (25) zum Ermitteln einer Pupillenkontur und einen Modellierer umfassen, um eine Funktion zur Annäherung an die Pupillenkontur zu bestimmen.

**12.** Pupillometer gemäß Anspruch 11, **dadurch gekennzeichnet, dass** die Näherungsfunktion eine Funktion zweiter Ordnung ist.

## Revendications

**1.** Pupillomètre automatique, comprenant :

une chambre à l'abri de la lumière (2) pouvant être connectée à au moins un oeil (8) d'un patient ;
un dispositif d'éclairage (3) pour fournir des stimuli lumineux ayant des valeurs d'intensité respectives sélec-tionnables ($I_1$, $I_2$, ..., $I_N$) à l'intérieur de la chambre (2) ;
un moyen de mesure de la réponse pupillaire (7), pour déterminer les valeurs d'au moins un diamètre pupillaire ($D_1$, $D_2$, ..., $D_N$), lesdites valeurs correspondant aux valeurs d'intensité respectives choisies ($I_1$, $I_2$, ..., $I_N$) ;
**caractérisé en ce que** le moyen de mesure de la réponse pupillaire (7) comprend un moyen de stockage (16) pour stocker un groupe de coefficients ($T_1$, $T_2$, ..., $T_N$) associés aux valeurs d'intensité respectives ($I_1$, $I_2$, ..., $I_N$) et un moyen de calcul (17), pour calculer un diamètre pupillaire optimal ($D_X$) sur la base des valeurs du diamètre pupillaire ($D_1$, $D_2$, ..., $D_N$) et des coefficients stockés ($T_1$, $T_2$, ..., $T_N$).

**2.** Pupillomètre selon la revendication 1, **caractérisé en ce que** ladite valeur optimale ($D_x$) satisfait à la condition :

$$(100 - P)\sum_{i=1}^{K} T_i(D_X - D_i) = P \sum_{j=K+1}^{N} T_j(D_j - D_X)$$

$$D_x \in \left[D_K, D_{K+1}\right]$$

dans laquelle N est le nombre de stimuli lumineux générés par le dispositif d'éclairage (3), K est un nombre entier compris entre 2 et N-1 et P est un coefficient en pourcentage entre 0 et 100.

**3.** Pupillomètre selon la revendication 1, **caractérisé en ce que** ledit diamètre pupillaire optimal ($D_X$) est en corrélation avec une moyenne pondérée desdites valeurs du diamètre pupillaire ($D_1$, $D_2$, ..., $D_N$), sur la base des coefficients stockés ($T_1$, $T_2$, ..., $T_N$).

**4.** Pupillomètre selon l'une quelconque des revendications précédentes, **caractérisé en ce que** lesdits coefficients ($T_1$, $T_2$, ..., $T_N$) sont en corrélation avec les temps de séjour moyens du patient dans des conditions d'éclairage correspondant aux valeurs d'intensité respectives ($I_1$, $I_2$, ..., $I_N$) des stimuli lumineux.

5. Pupillomètre selon l'une quelconque des revendications précédentes, **caractérisé en ce que** ledit moyen de mesure de la réponse pupillaire (7) comprend un moyen d'acquisition d'image (12, 13), pour acquérir une image respective de l'oeil (8) pour chaque valeur d'intensité choisie ($I_1$, $I_2$, ..., $I_N$), et un moyen de traitement d'image (15), associé au moyen d'acquisition d'image (12, 13) pour déterminer les valeurs dudit diamètre ($D_1$, $D_2$, ..., $D_N$) de la pupille (19).

6. Pupillomètre selon l'une quelconque des revendications précédentes, **caractérisé en ce qu'**il comprend un moyen de contrôle (6) du dispositif d'éclairage, pour contrôler les valeurs d'intensité ($I_1$, $I_2$, ..., $I_N$) des stimuli lumineux.

7. Pupillomètre selon la revendication 6, **caractérisé en ce que** le moyen de contrôle (6) comprend une unité de sélection (20) pour sélectionner lesdites valeurs d'intensité ($I_1$, $I_2$, ..., $I_N$) des stimuli lumineux.

8. Pupillomètre selon la revendication 7, **caractérisé en ce qu'**il comprend un capteur (5) de l'intensité lumineuse, fournissant un signal de mesure ($I_M$) en corrélation avec une valeur d'intensité existante du rayonnement lumineux incident sur le capteur (5), et **en ce que** le moyen de contrôle (6) comprend une étape de comparaison (21) fournissant un signal d'erreur ($S_E$) en corrélation avec une différence entre le signal de mesure ($I_M$) et la valeur d'intensité sélectionnée ($I_1$, $I_2$, ..., $I_N$), et une unité de commande (22), associée avec ledit dispositif d'éclairage (3) pour modifier la valeur d'intensité ($I_1$, $I_2$, ..., $I_N$) des stimuli lumineux sur la base du signal d'erreur ($S_E$), de manière à annuler le signal d'erreur ($S_E$).

9. Pupillomètre selon la revendication 8 dépendante de la revendication 5, **caractérisé en ce que** le capteur d'intensité lumineuse (5) est intégré dans ladite chambre (2).

10. Pupillomètre selon l'une quelconque des revendications précédentes, **caractérisé en ce que** ledit dispositif d'éclairage (3) comprend une source de lumière diffusée (10) et une source de lumière directe (11).

11. Pupillomètre selon l'une quelconque des revendications 5 à 10,
**caractérisé en ce que** ledit moyen de traitement d'image (15) comprend au moins un filtre binaire (24), une unité de détection de contour (25), pour identifier un contour pupillaire, et un dispositif de modélisation (27) pour déterminer une fonction d'approximation du contour pupillaire.

12. Pupillomètre selon la revendication 11, **caractérisé en ce que** la fonction d'approximation est une fonction de second ordre.

Fig.1

Fig.2

Fig.3

**Fig.4**

**Fig.5**

$$D_1, D_2, ..., D_N$$

**Fig.6**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• EP 1127534 A